# EUROPÄISCHE PATENTSCHRIFT

(11) **EP 0 594 539 B1**
(45) Veröffentlichungstag und Bekanntmachung des Hinweises auf die Patenterteilung: **02.05.1997**
(21) Anmeldenummer: 93810717.4
(22) Anmeldetag: 12.10.1993
(51) Int. Cl.: C07F 9/6574, C08K 5/527, C07C 39/23, C08K 5/13

(54) **Phenylphosphite als Stabilisatoren für organische Materialien**
Phenyl phosphites as stabilizers for organic materials
Phosphites de phényle comme stabilisants pour matériaux organiques

(30) Priorität: 21.10.1992 CH 3262/92
(43) Veröffentlichungstag der Anmeldung: 27.04.1994
(73) Patentinhaber: Ciba Specialty Chemicals Holding Inc., 4057 Basel (CH)
(72) Erfinder: Nesvadba, Peter, Dr., CH-1723 Marly (CH)

(56) Entgegenhaltungen:
- EP-A- 0 048 878
- GB-A- 2 027 713
- US-A- 3 281 381
- JOURNAL OF THE AMERICAN CHEMICAL SOCIETY Bd. 106, Nr. 4 , 22. Februar 1984 , WASHINGTON, DC US Seiten 1132 - 1133 PENG-CHO TANG 'Cyclohexadienone annulation via alpha,beta-unsaturated Fischer carbene complexes'

## Beschreibung

Die vorliegende Erfindung betrifft neue Phenylphosphite, Zusammensetzungen, enthaltend ein organisches Material, bevorzugt ein Polymer, und die neuen Phenylphosphite, sowie die Verwendung derselben zum Stabilisieren von organischen Materialien gegen oxidativen, thermischen oder lichtinduzierten Abbau.

Organische Phosphite sind in der Technik als Costabilisatoren, sekundäre Antioxidantien und Verarbeitungsstabilisatoren, unter anderem für Polyolefine, bekannt; Beispiele für solche bekannten Phosphitstabilisatoren finden sich in R. Gächter/H. Müller (Ed.), Plastics Additives Handbook, 3rd Ed., p. 47, Hanser, München 1990, und in EP-A-356 688.

US-A-3 281 381 offenbart Reaktionsprodukte von beispielsweise Pentaerythrit mit Triarylphosphiten als PVC-Stabilisatoren. GB-A-2 027 713 offenbart die Verwendung von Triarylphosphiten als Stabilisatoren für Polyethylen.

Es besteht weiterhin ein Bedarf an wirksamen Stabilisatoren für organische Materialien, die gegen oxidativen, thermischen oder lichtinduzierten Abbau empfindlich sind.

Es wurde nun gefunden, dass eine ausgewählte Gruppe solcher Phosphite sich überraschenderweise besonders gut als Stabilisatoren für organische Materialien, die gegen oxidativen, thermischen oder lichtinduzierten Abbau empfindlich sind, eignen. Insbesondere hervorzuheben ist die Eignung der genannten Verbindungen als Verarbeitungsstabilisatoren für synthetische Polymere.

Die vorliegende Erfindung betrifft daher Verbindungen der Formel I oder II 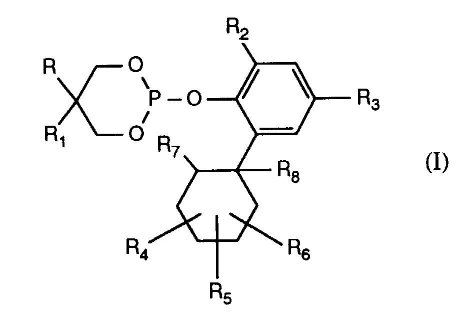 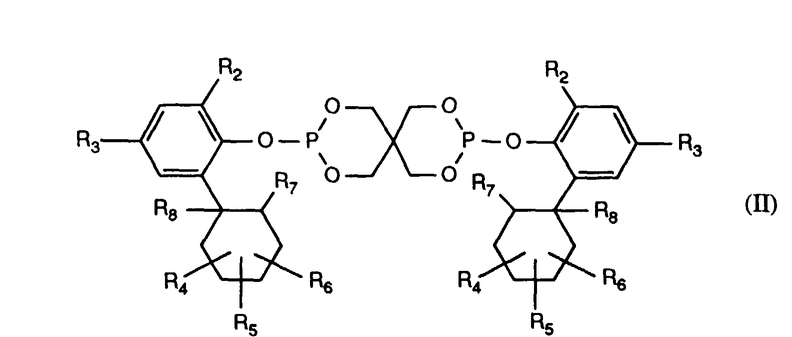 worin
R und R₁ unabhängig voneinander Wasserstoff oder C₁-C₄-Alkyl bedeuten oder zusammen mit dem Kohlenstoffatom, an das sie gebunden sind, einen 3,4-Dehydrocyclohexylidenring bilden,
R₂ Wasserstoff, C₁-C₈-Alkyl oder C₅-C₆-Cycloalkyl darstellt,
R₃ C₁-C₈-Alkyl oder C₅-C₆-Cycloalkyl bedeutet,
R₄, R₅ und R₆ unabhängig voneinander Wasserstoff oder C₁-C₄-Alkyl sind, wobei R₄, R₅ und R₆ zusammen 1 bis 4 Kohlenstoffatome enthalten, und
R₇ und R₈ Wasserstoff bedeuten oder gemeinsam eine zusätzliche direkte Bindung bilden.

Alkyl mit bis zu 8 Kohlenstoffatomen bedeutet einen verzweigten oder unverzweigten Rest wie beispielsweise Methyl, Ethyl, Propyl, Isopropyl, n-Butyl, sec-Butyl, Isobutyl, tert-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methyl-heptyl, n-Octyl oder 2-Ethylhexyl. Für R und R₁ ist Methyl, für R₂ und R₃ ist tert-Butyl bevorzugt.

Der 3,4-Dehydrocyclohexylidenring bedeutet 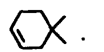 C₅-C₆-Cycloalkyl bedeutet beispielsweise Cyclopentyl oder Cyclohexyl.

Falls R₄, R₅ und R₆ unabhängig voneinander Wasserstoff oder C₁-C₄-Alkyl sind, wobei R₄, R₅ und R₆ zusammen 1 bis 4 Kohlenstoffatome enthalten, handelt es sich beispielsweise um folgende Cyclohexen-1-yl-Substituenten an den Phenylresten in den allgemeinen Formeln I oder II:

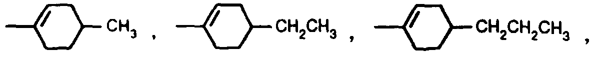

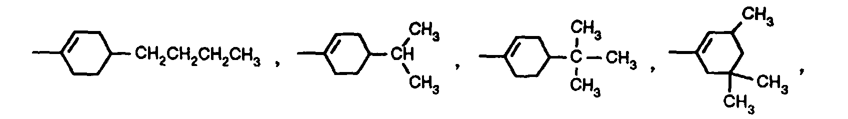

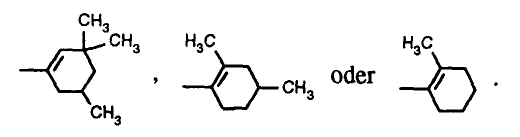

Bevorzugt sind die Verbindungen der Formel I oder II, worin
R₂ Wasserstoff oder C₁-C₄-Alkyl bedeutet,
R₃ C₁-C₄-Alkyl darstellt,
R₄, R₅ und R₆ Wasserstoff sind, und
R₇ und R₈ gemeinsam eine zusätzliche direkte Bindung bilden.

Besonders bevorzugt sind die Verbindungen der Formel I oder II, worin R und R₁ Methyl sind, R₂ und R₃ C₁-C₄-Alkyl bedeuten, und R₄, R₅ und R₆ Wasserstoff darstellen.

Von besonderem Interesse sind die Verbindungen der Formel I oder II, worin R₇ und R₈ gemeinsam eine zusätzliche direkte Bindung bilden.

Die erfindungsgemässen Verbindungen der Formel I und Formel II können auf an sich bekannte Weise hergestellt werden.

Beispielsweise, und dies ist bevorzugt, wird ein Phenol der Formel III 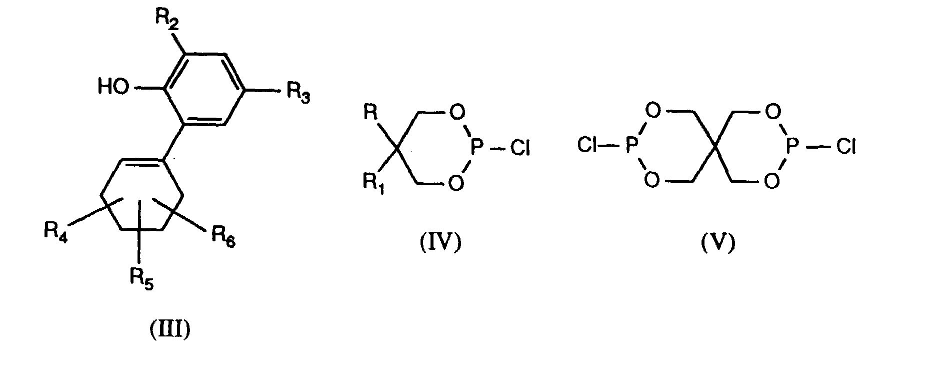 mit einem Chorphosphit der Formel IV oder V in Gegenwart eines geeigneten organischen, polaren oder apolaren, aprotischen Lösungsmittels umgesetzt. Bevorzugt geschieht diese Umsetzung in Gegenwart einer Base bei Temperaturen zwischen -20°C und dem Siedepunkt des Lösungsmittels.

Die Base kann in unterschiedlichen Mengen eingesetzt werden, von katalytischen über stöchiometrische Mengen bis hin zu mehrfachem molarem Überschuß bezüglich eingesetztem Phenol oder Chlorphosphit. Der bei der Reaktion gebildete Chlorwasserstoff wird gegebenenfalls durch die Base in Chlorid überführt, das durch Filtration und/oder Waschen mit einer geeigneten wässrigen oder festen Phase entfernt werden kann; dabei kann auch ein zweites, nicht mit Wasser mischbares Lösungsmittel eingesetzt werden. Die Reinigung des Produktes erfolgt zweckmäßig durch Umkristallisation des Rückstandes der eingeengten oder zur Trockne eingedampften organischen Phase.

Geeignete Lösungsmittel zur Durchführung der Reaktion sind u.a. Kohlenwasserstoffe (beispielsweise Mesitylen, Toluol, Xylol, Hexan, Pentan oder weitere Petroletherfraktionen), halogenierte Kohlenwasserstoffe (beispielsweise Di- oder Trichlormethan, 1,2-Dichlorethan, 1,1,1-Trichlorethan), Ether (z.B. Diethylether, Dibutylether oder Tetrahydrofuran), ferner Acetonitril, Dimethylformamid, Dimethylsulfoxid oder N-Methylpyrrolidon.

Geeignete Basen sind u.a. tertiäre Amine (z.B. Trimethylamin, Triethylamin, Tributylamin, N,N-Dimethylanilin, N,N-Diethylanilin oder Pyridin), Hydride (z.B. Lithium-, Natrium-, Kaliumhydrid) oder Alkoholate (z.B. Natriummethanolat).

Hydride (z.B. Natriumhydrid, Natriumborhydrid oder Lithiumaluminiumhydrid), Alkalimetalle, Alkalihydroxide oder Natriummethanolat können auch zur Bildung des Phenolats der Formel III verwendet werden; das gegebenenfalls dabei entstehende Reaktionsprodukt (z.B. Wasser, Methanol) wird vor der Umsetzung mit dem Chlorphosphit der Formel IV oder V abdestilliert (z.B. als Azeotrop mit Toluol).

Die Herstellung der Chlorphosphite der Formel IV und V ist bekannt und beispielsweise in Houben-Weyl, Band E1, Seite 373-376, Georg Thieme Verlag, Stuttgart, 1982, und Houben-Weyl, 2. Auflage, Band XII/2, Seite 48, Georg Thieme Verlag, Stuttgart 1964, beschrieben.

Die erfindungsgemässen Verbindungen der Formel I und Formel II, worin R₇ und R₈ gemeinsam eine zusätzliche direkte Bindung bilden, können beispielsweise durch katalytische Hydrierung analog Organikum, Seiten 288-298, Deutscher Verlag der Wissenschaften Berlin 1986, in einem organischen Lösungsmittel in einem Temperaturbereich von z.B. 0 bis 100°C und eventuell unter leichtem Druck zu den erfindungsgemässen Verbindungen der Formel I und Formel II, worin R₇ und R₈ Wasserstoff bedeuten, umgesetzt werden. Als Katalysatoren werden bevorzugt Palladium auf Kohle, Platinoxid oder Raney-Nickel verwendet. Als organische Lösungsmittel, die auch Wasser enthalten dürfen, eignen sich besonders Alkohole, wie beispielsweise Methanol, Ethanol oder Isopropanol, sowie Tetrahydrofuran, Dioxan, Esssigester, Toluol oder Dimethylacetamid.

Die erfindungsgemässen Verbindungen der Formel I und Formel II, worin R₇ und R₈ Wasserstoff bedeutet, können ebenfalls ausgehend vom hydrierten Phenol der Formel IIIa 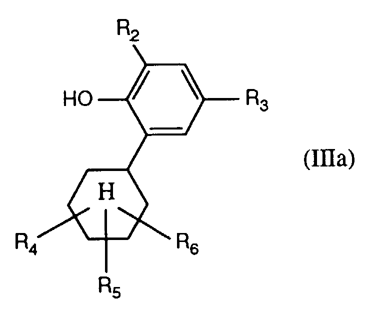 welches beispielsweise durch katalytische Hydrierung der Doppelbindung analog den oben aufgeführten Reaktionsbedingungen ausgehend vom Phenol der Formel III hergestellt wird, mit einem Chlorphosphit der Formel IV oder V analog den oben aufgeführten Reaktionsbedingungen in Gegenwart eines geeigneten organischen, polaren oder apolaren Lösungsmittels hergestellt werden.

Die Phenole der Formel III sind in der Literatur weitgehend unbekannt. Einzig die Verbindung der Formel VI ist von W.D. Wulff et al, J. Amer. Chem. Soc. 106, 1132 (1984) auf eine komplizierte Art hergestellt worden.

Die vorliegende Erfindung betrifft daher auch Verbindungen der Formel III 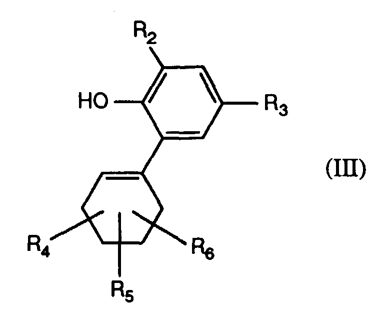 worin
R₂ Wasserstoff, C₁-C₈-Alkyl oder C₅-C₆-Cycloalkyl darstellt,
R₃ C₁-C₈-Alkyl oder C₅-C₆-Cycloalkyl bedeutet,
R₄, R₅ und R₆ unabhängig voneinander Wasserstoff oder C₁-C₄-Alkyl sind, wobei R₄, R₅ und R₆ zusammen 1 bis 4 Kohlenstoffatome enthalten, mit der Bedingung, dass die Verbindung der Formel VI ausgeschlossen ist.

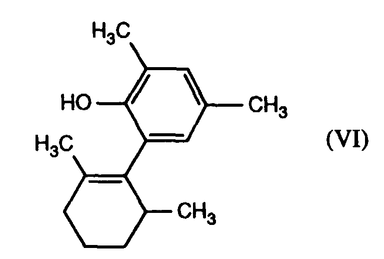

Bevorzugt sind Verbindungen der Formel III, worin
R₂ Wasserstoff oder C₁-C₄-Alkyl bedeutet,
R₃ C₁-C₄-Alkyl darstellt, und
R₄, R₅ und R₆ Wasserstoff sind.

Besonders bevorzugt sind Verbindungen der Formel III, worin
R₂ tert-Butyl bedeutet.

Die Herstellung der Verbindungen der Formel III erfolgt beispielsweise, und dies ist bevorzugt, durch Kondensation der mindestens in einer ortho-Position unsubstituierten Phenole der Formel VII 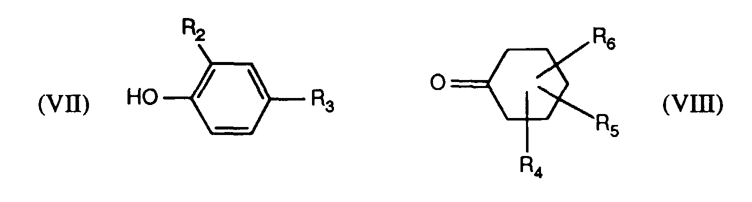 mit Cyclohexanonen der Formel VIII. Die Reaktion wird bei erhöhter Temperatur, insbesondere Temperaturen von 20 bis 100°C in der Schmelze oder in einem Lösungsmittel, gegebenenfalls unter leichtem Druck, durchgeführt. Bevorzugt wird die Reaktion in der Schmelze in einem Temperaturbereich von 20 bis 80°C, insbesondere 40 bis 60°C, durchgeführt. Die Reaktion kann durch Zusatz einer Säure wie Salzsäure, Schwefelsäure oder Methansulfonsäure katalysiert werden. Bevorzugt wird Salzsäure-Gas verwendet.

Die erfindungsgemässen Verbindungen der Formel I und Formel II eignen sich hervorragend zum Stabilisieren von organischen Materialien gegen oxidativen, thermischen oder lichtinduzierten Abbau. Auch die Verbindungen der Formel m eignen sich zum Stabilisieren von organischen Materialien gegen oxidativen, thermischen oder lichtinduzierten Abbau.

Beispiele für solche organische Materialien [Komponente (a)] sind:
1. Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE), verzweigtes Polyethylen niederer Dichte (VLDPE).
   Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:
   a) radikalisch (gewöhnlich bei hohem Druck und hoher Temperatur).
   b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder π- oder σ-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metallocen oder Single Site Katalysatoren (SSC) bezeichnet.
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.
4. Kohlenwasserstoffharze (z.B. C₅-C₉) inklusive hydrierte Modifikationen davon (z.B. Klebrigmacherharze) und Mischungen von Polyalkylenen und Stärke.
5. Polystyrol, Poly-(p-methylstyrol), Poly-(α-methylstyrol).
6. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
7. Pfropfcopolymere von Styrol oder α-Methylstyrol, wie z.B. Styrol auf Polybutadien, Styrol auf Polybutadien- Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.
8. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen,wie z.B. Polyvinylchlorid, Polyvinylidenchlorid, Polyvinylfluorid, vinylidenfluorid; sowie deren Copolymere, wie Vinylchlorid-Vinylidenchlorid, Vinylchlorid-Vinylacetat oder Vinylidenchlorid-Vinylacetat.
9. Polymere, die sich von α,β-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.
10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-VinylhalogenidCopolymere oder Acrylnitril-Alkylmethacrylat-Butadien-Terpolymere.
11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
12. Homo- und Copolymere von cyclischen Ethern, wie Polyalkylenglykole, Polyethylenoxyd, Polypropylenoxyd oder deren Copolymere mit Bisglycidylethern.
13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
14. Polyphenylenoxide und -sulfide und deren Mischungen mit Styrolpolymeren oder Polyamiden.
15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
17. Polyharnstoffe, Polyimide, Polyamid-imide und Polybenzimidazole.
18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
19. Polycarbonate und Polyestercarbonate.
20. Polysulfone, Polyethersulfone und Polyetherketone.
21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
22. Trocknende und nicht-trocknende Alkydharze.
23. Ungesättigte Polyesterharze, die sich von Copolyestern gsättigter und ungesättigter Di-carbonsäuren mit mehrwertigen Alkoholen, sowie Vinylverbindungen als Vernetzungsmittel ableiten, wie auch deren halogenhaltige, schwerbrennbare Modifikationen.
24. Vernetzbare Acrylharze, die sich von substituierten Acrylsäureestern ableiten, wie z.B. von Epoxyacrylaten, Urethan-acrylaten oder Polyester-acrylaten.
25. Alkydharze, Polyesterharze und Acrylatharze, die mit Melaminharzen, Harnstoffharzen, Polyisocyanaten oder Epoxidharzen vernetzt sind.
26. Vernetzte Epoxidharze, die sich von Polyepoxiden ableiten, z.B. von Bis-glycidylethern oder von cycloaliphatischen Diepoxiden.
27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.
29. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.
30. Wässrige Emulsionen natürlicher oder synthetischer Kautschuke, wie z.B. Naturkautschuk-Latex oder Latices von carboxylierten Styrol-Butadien-Copolymeren.

Weitere Gegenstände der Erfindung sind daher auch Zusammensetzungen enthaltend (a) ein dem oxidativen, thermischen oder lichtinduzierten Abbau unterworfenes organisches Material und (b) mindestens eine Verbindung der Formel I oder Formel II.

Ebenfalls Gegenstand der Erfindung ist eine Zusammensetzung enthaltend (a) ein dem oxidativen, thermischen oder lichtinduzierten Abbau unterworfenes organisches Material und (b) mindestens eine Verbindung der Formel III.

Vorzugsweise handelt es sich bei den zu schützenden organischen Materialien um natürliche, halbsynthetische oder bevorzugt synthetische organische Materialien. Besonders bevorzugt sind thermoplastische Polymere, insbesondere PVC oder Polyolefine, vor allem Polyethylen und Polypropylen.

Besonders hervorzuheben ist die Wirkung der erfindungsgemässen Verbindungen gegen thermischen und oxidativen Abbau, vor allem bei thermischer Belastung, wie sie bei der Verarbeitung von Thermoplasten auftritt. Die erfindungsgemässen Verbindungen sind daher hervorragend als Verarbeitungsstabilisatoren einzusetzen.

Vorzugsweise werden die Verbindungen der Formel I oder Formel II dem zu stabilisierenden Material in Mengen von 0,01 bis 10 %, beispielsweise 0,01 bis 5 %, vorzugsweise 0,05 bis 3 %, insbesondere 0,05 bis 1 % zugesetzt, bezogen auf das Gewicht des zu stabilisierenden organischen Materials.

Zusätzlich zu den Verbindungen der Formel I oder Formel II können die erfindungsgemässen Zusammensetzungen weitere Costabilisatoren enthalten, wie beispielsweise die folgenden:
1. Antioxidantien
   1.1. Alkylierte Monophenole, z.B. 2,6-Di-tert-butyl-4-methylphenol, 2-butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6 2,6-Di-tert-butyl-4-ethylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.
   1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.
   1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl4-hydroxyanisol, 3,5-Di-tert-butyl-4-hydroxyanisol, 3,5-Di-tert-butyl-4-hydroxyphenyl-stearat, Bis-(3,5-di-tert-butyl-4-hydroxyphenyl)adipat.
   1.4. Hydroxylierte Thiodiphenylether, z.B. 2,2'-Thio-bis-(6-tert-butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methylphenol), 4,4'-Thio-bis-(3,6-di-sec.-amylphenol), 4,4'-Bis-(2,6-dimethyl-4-hydroxyphenyl)-disulfid.
   1.5. Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methylphenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methyl-phenyl)-dicyclopentadien, Bis-[2-(3'-tert-butyl-2'-hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis-(3,5-di-tert-butyl-4-hydroxyphenyl)-propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.
   1.6. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)dithioterephthalat, Bis-(3,5-di-tert-butyl-4-hydroxybenzyl)-sulfid, Isooctyl-3,5-di-tert-butyl-4-hydroxybenzyl-mercaptoacetat.
   1.7. Hydroxybenzylierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis-(3,5-di-tert-butyl-4-hydroxybenzyl)-malonat, Di-[4-(1,1,3,3-tetramethylbutyl)-phenyl]-2,2-bis-(3,5-di-tert-butyl-4-hydroxybenzyl)-malonat.
   1.8. Hydroxybenzyl-Aromaten, z.B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.
   1.9. Triazinverbindungen, z.B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.
   1.10. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.
   1.11. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.
   1.12. Ester der β-(3,5-Di-tert-butyl-4-hydroxyphenyl)-propionsäure mit ein- oder mehr wertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethyl-hexandiol, Trimethylolpropan, 4-Hydroxymethyl-1-phospha-2,6,7-trioxabicyclo-[2.2.2]-octan.
   1.13. Ester der β-I(5-tert-Butyl-4-hydroxy-3-methylphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N' -Bis-(hydroxyethyl)-oxalsäurediamid, 3-Thiaundecanol, 3-Thiapentadecanol, Tri-methylhexandiol, Trimethylolpropan, 4-Hydroxymethyl-1-phospha-2,6,7-trioxabicyclo-[2.2.2]-octan.
   1.14. Ester der β-(3,5-Dicyclohexyl-4-hydroxyphenyl)-propionsäure mit ein- oder mehr wertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethyl-hexandiol, Trimethylolpropan, 4-Hydroxymethyl-1-phospha-2,6,7-trioxabicyclo-[2.2.2]-octan.
   1.15.Ester der 3,5-Di-tert-butyl-4-hydroxyphenylessigsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxy-ethyl)-oxalsäurediamid, 3-Thiaundecanol, 3-Thiapentadecanol, Trimethylhexandiol, Tri-methylolpropan, 4-Hydroxymethyl-1-phospha-2,6,7-trioxabicyclo-[2.2.2]-octan.
   1.16. Amide der β-(3,5-Di-tert-butyl-4-hydroxyphenyl)-propionsäure, wie z.B. N,N'-Bis(3,5-di-tert-butyl-4-hydroxyphenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.
2. UV-Absorber und Lichtschutzmittel
   2.1.2-(2'-Hydroxyphenyl)-benztriazole, wie z.B. 2-(2'-Hydroxy-5'-methylphenyl)-benztriazol, 2-(3',5'-Di-tert-butyl-2'-hydroxyphenyl)-benztriazol, 2-(5'-tert-Butyl-2'-hydroxy-phenyl)-benztriazol, 2-(2'-Hydroxy-5'-(1,1,3,3-tetramethylbutyl)phenyl)-benztriazol, 2-(3',5'-Di-tert-butyl-2'-hydroxyphenyl)-5-chlor-benztriazol, 2-(3'-tert-Butyl-2'-hydroxy-5'-methylphenyl)-5-chlor-benztriazol, 2-(3'-sec-Butyl-5'-tert-butyl-2'-hydroxyphenyl)-benztriazol, 2-(2'-Hydroxy-4'-octoxyphenyl)-benztriazol, 2-(3',5'-Di-tert-amyl-2'-hydroxyphenyl)-benztriazol, 2-(3',5'-Bis-(α,α-dimethylbenzyl)-2'-hydroxyphenyl)-benztriazol, Mischung aus 2-(3'-tert-Butyl-2'-hydroxy-5'-(2-octyloxycarbonylethyl)-phenyl)-5-chlor-benztriazol, 2-(3'-tert-Butyl-5'-[2-(2-ethylhexyloxy)-carbonylethyl]-2'-hydroxyphenyl)-5-chlor-benztriazol, 2-(3'-tert-Butyl-2'-hydroxy-5'-(2-methoxycarbonyl-ethyl)phenyl)-5-chlor-benztriazol, 2-(3'-tert-Butyl-2'-hydroxy-5'-(2-methoxycarbonyl-ethyl)phenyl)-benztriazol, 2-(3'-tert-Butyl-2'-hydroxy-5'-(2-octyloxycarbonylethyl)-phenyl)-benztriazol, 2-(3'-tert-Butyl-5'-[2-(2-ethylhexyloxy)carbonylethyl]-2'-hydroxy-phenyl)-benztriazol, 2-(3'-Dodecyl-2'-hydroxy-5'-methylphenyl)-benztriazol, und 2-(3'-tert-Butyl-2'-hydroxy-5'-(2-isooctyloxycarbonylethyl)phenyl-benztriazol, 2,2'-Methylen-bis[4-(1,1,3,3-tetramethylbutyl)-6-benztriazol-2-yl-phenol]; Umesterungsprodukt von 2-[3'-tert-Butyl-5'-(2-methoxycarbonylethyl)-2'-hydroxy-phenyl]-benztriazol mit Polyethylenglycol 300;[R-CH₂CH₂-COO(CH₂)₃ 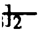 mit R =3'-tert-Butyl-4'-hydroxy-5'-2H-benztriazol-2-yl-phenyl.
   2.2. 2-Hydroxybenzoyhenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.
   2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.
   2.4. Acrylate, wie z.B. α-Cyan-β,β-diphenylacrylsäure-ethylester bzw. -isooctylester, a-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.
   2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.
   2.6. Sterisch gehinderte Amine, wie z.B. Bis-(2,2,6,6-tetramethyl-piperidyl)-sebacat, Bis-(2,2,6,6-tetramethyl-piperidyl)-succinat, Bis-(1,2,2,6,6-pentamethylpiperidyl)-sebacat, n-Butyl-3,5-di-ten-butyl-4-hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert-Octylamino-2,6-dichlor-1,3,5-s-triazin, Tris-(2,2,6,6-tetramethyl- 4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon), 4-Benzoyl-2,2,6,6-tetramethylpiperidin, 4-Stearyloxy-2,2,6,6-tetramethylpiperidin, Bis(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis-(1-octyloxy-2,2,6,6-tetramethylpiperidyl)-sebacat, Bis-(1-octyloxy-2,2,6,6-tetramethylpiperidyl)succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino)-2,2,6,6-tetramethylpiperidyl)-1,3,5-triazin und 1,2-Bis(3-aminopropylamino)äthan, Kondensationsprodukt aus 2-Chlor-4,6-di-(4-n-butylamino1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion.
   2.7. Oxalsäurediamide, wie z.B. 4,4'-Di-octyloxy-oxanilid, 2,2'-Di-octyloxy-5,5'-di-tert-butyl-oxanilid, 2,2'-Di-dodecyloxy-5,5'di-tert-butyl-oxanilid, 2-Ethoxy-2'-ethyl-oxanilid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.
   2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.
3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert-butyl-4-hydroxyphenylpropionyl)-hydrazin, 3-Salicyloylamino-1,2,4-triazol, Bis-(benzyliden)-oxalsäuredihydrazid, Oxanilid, Isophthalsäure-dihydrazid, Sebacinsäure-bis-phenylhydrazid, N,N'-Diacetyl-adipinsäure-dihydrazid, N,N'-Bis-salicyloyl-oxalsäure-dihydrazid, N,N'-Bis-salicyloyl-thiopropionsäure-dihydrazid.
4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythrit-diphosphit, Bis-(2,4-di-tert-butylphenyl)-pentaerythritdiphosphit, Bis(2,6-di-tert-butyl-4-methylphenyl)-pentaerythritdiphosphit, Bis-isodecyloxy-pentaerythritdiphosphit, Bis-(2,4-di-tert-butyl-6-methylphenyl)-pentaerythritdiphosphit, Bis-(2,4,6-tri-tert-butylphenyl)-pentaerythlitdiphosphit, Tristearyl-sorbit-triphosphit, Tetrakis-(2,4-di-tert-butylphenyl)-4,4'-biphenylen-diphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-di-benz[d,g]-1,3,2-dioxaphosphocin, Bis-(2,4-di-tert-butyl-6-methylphenyl)-methylphosphit, Bis-(2,4-di-tert-butyl-6-methylphenyl)-ethylphosphit.
5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.
6. Polyamidstabilisatoren, wie z.B. Kupfersalze in Kombination mit Jodiden und/oder Phosphorverbindungen und Salze des zweiwertigen Mangans.
7. Basische Co-Stabilisatoren, wie z.B. Melamin, Polyvinylpyrrolidon, Dicyandiamid, Tri-allylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.
8. Nukleierungsmittel, wie z.B. 4-tert-Butylbenzoesäure, Adipinsäure, Diphenylessigsäure.
9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Ruß, Graphit.
10. Sonstige Zusätze, wie z.B. Weichmacher, Gleitmittel, Emulgatoren, Pigmente, Optische Aufheller, Flammschutzmittel, Antistatika, Treibmittel.
11. Benzofuranone bzw. Indolinone, wie z.B. in US-A-4 325 863, US-A-4 338 244 oder US-A-5 175 312 beschrieben, oder 3-[4-(2-Acetoxyethoxy)phenyl]-5,7-di-tert-butyl-benzofuran-2-on.

Die Costabilisatoren, mit Ausnahme der unter Punkt 11 aufgeführten Benzofuranone, werden beispielsweise in Konzentrationen von 0,01 bis 10 %, bezogen auf das Gesamtgewicht des zu stabilisierenden Materials, zugesetzt.

Weitere bevorzugte Zusammensetzungen enthalten neben der Komponente (a) und den Verbindungen der Formel I oder Formel II noch weitere Additive, insbesondere phenolische Antioxidantien, Lichtschutzmittel oder Verarbeitungsstabilisatoren.

Besonders bevorzugte Additive sind phenolische Antioxidantien (Punkt 1 der Liste), sterisch gehinderte Amine (Punkt 2.6 der Liste), Phosphite und Phosphonite (Punkt 4. der Liste) und peroxidzerstörende Verbindungen (Punkt 5. der Liste).

Ebenfalls besonders bevorzugte zusätzliche Additive (Stabilisatoren) sind Benzofuran-2-one, wie sie z.B. in US-A-4 325 863, US-A-4 338 244 oder US-A-5 175 312 beschrieben werden.

Beispiele für solche Benzofuran-2-one sind Verbindungen der Formel 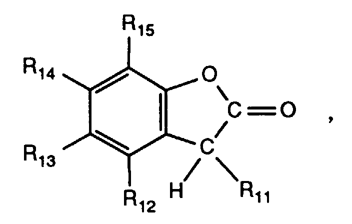 worin
R₁₁ Phenyl oder durch 1 bis 3 Alkylreste mit zusammen höchstens 18 Kohlenstoffatomen, Alkoxy mit 1 bis 12 Kohlenstoffatomen, Alkoxycarbonyl mit 2 bis 18 Kohlenstoffatomen oder Chlor substituiertes Phenyl ist; R₁₂ Wasserstoff ist;
R₁₄ Wasserstoff, Alkyl mit 1 bis 12 Kohlenstoffatomen, Cyclopentyl, Cyclohexyl oder Chlor ist;
R₁₃ die Bedeutung von R₁₂ oder R₁₄ hat oder ein Rest der Formel 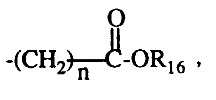 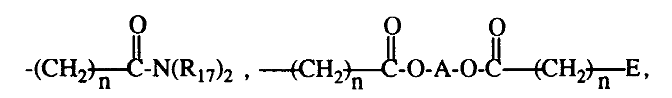 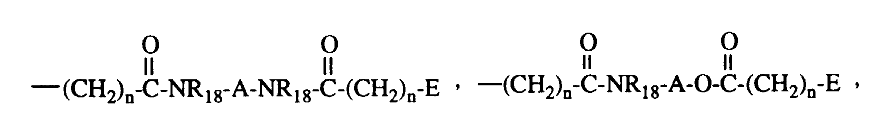 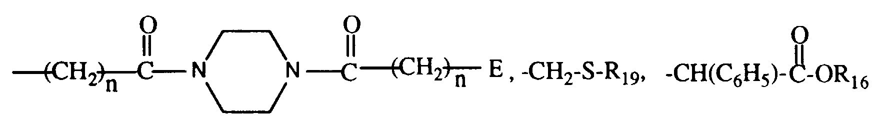 oder -D-E ist, worin
R₁₆ Wasserstoff, Alkyl mit 1 bis 18 Kohlenstoffatomen, durch Sauerstoff oder Schwefel unterbrochenes Alkyl mit 2 bis 18 Kohlenstoffatomen, Dialkylaminoalkyl mit insgesamt 3 bis 16 Kohlenstoffatomen, Cyclopentyl, Cyclohexyl, Phenyl oder durch 1 bis 3 Alkylreste mit zusammen höchstens 18 Kohlenstoffatomen substituiertes Phenyl ist;
n 0, 1 oder 2 ist;
die Substituenten R₁₇ unabhängig voneinander Wasserstoff, Alkyl mit 1 bis 18 Kohlenstoffatomen, Cyclopentyl, Cyclohexyl, Phenyl, durch 1 oder 2 Alkylreste mit zusammen höchstens 16 Kohlenstoffatomen substituiertes Phenyl, ein Rest der Formel -C₂H₄OH, -C₂H₄-O-CₘH₂ₘ₊₁ oder 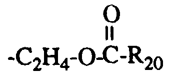 sind oder zusammen mit dem Stickstoffatom, an das sie gebunden sind, einen Piperidin- oder Morpholinrest bilden;
m 1 bis 18;
R₂₀ Wasserstoff, Alkyl mit 1 bis 22 Kohlenstoffatomen oder Cycloalkyl mit 5 bis 12 Kohlenstoffatomen;
A ein gegebenenfalls durch Stickstoff, Sauerstoff oder Schwefel unterbrochenes Alkylen mit 2 bis 22 Kohlenstoffatomen;
R₁₈ Wasserstoff, Alkyl mit 1 bis 18 Kohlenstoffatomen, Cyclopentyl, Cyclohexyl, Phenyl, durch 1 oder 2 Alkylreste mit zusammen höchstens 16 Kohlenstoffatomen substituiertes Phenyl oder Benzyl;
R₁₉ Alkyl mit 1 bis 18 Kohlenstoffatomen bedeutet;
D -O-, -S-, -SO-, -SO₂- oder -C(R₂₁)₂- ist;
die Substituenten R₂₁ unabhängig voneinander Wasserstoff, C₁-C₁₆-Alkyl sind, wobei die beiden R₂₁ zusammen 1 bis 16 Kohlenstoffatome enthalten, R₂₁ ferner Phenyl oder einen Rest der Formel 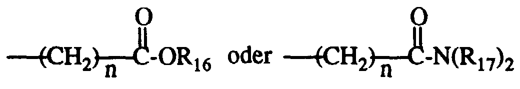 ist, worin n, R₁₆ und R₁₇ die oben angegebenen Bedeutungen haben;
E ein Rest der Formel 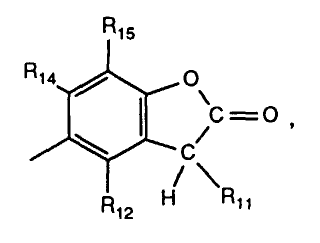 worin R₁₁, R₁₂ und R₁₄ die oben angegebenen Bedeutungen haben; und
R₁₅ Wasserstoff, Alkyl mit 1 bis 20 Kohlenstoffatomen, Cyclopentyl, Cyclohexyl, Chlor oder ein Rest der Formel 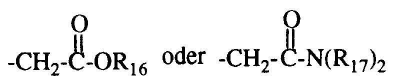 ist, worin R₁₆ und R₁₇ die oben angegebenen Bedeutungen haben, oder R₁₅ zusammen mit R₁₄ einen Tetramethylenrest bildet.

Bevorzugt sind solche Benzofuran-2-one, in denen R₁₃ Wasserstoff, Alkyl mit 1 bis 12 Kohlenstoffatomen, Cyclopentyl, Cyclohexyl, Chlor oder ein Rest der Formel 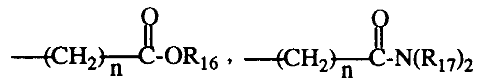 oder -D-E ist, worin n, R₁₆, R₁₇, D und E die oben angegebenen Bedeutungen haben, R₁₆ insbesondere die Bedeutung von Wasserstoff, Alkyl mit 1 bis 18 Kohlenstoffatomen, Cyclopentyl oder Cyclohexyl hat.

Bevorzugt sind weiterhin solche Benzofuran-2-one, in denen R₁₁ Phenyl oder durch 1 oder 2 Alkylreste mit zusammen höchstens 12 Kohlenstoffatomen substituiertes Phenyl ist; R₁₂ Wasserstoff; R₁₄ Wasserstoff oder Alkyl mit 1 bis 12 Kohlenstoffatomen ist; R₁₃ Wasserstoff, Alkyl mit 1 bis 12 Kohlenstoffatomen, 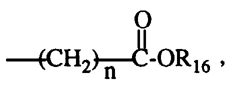 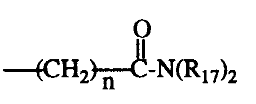 oder -D-E; R₁₅ Wasserstoff, Alkyl mit 1 bis 20 Kohlenstoffatomen, 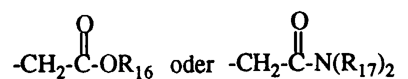 ist oder R₁₅ zusammen mit R₁₄ einen Tetramethylenrest bildet, wobei n, R₁₆, R₁₇, D und E die zu Anfang angegebenen Bedeutungen haben.

Ebenfalls von besonderem Interesse sind solche Benzofuran-2-one, in denen R₁₁ Phenyl; R₁₃ Wasserstoff, Alkyl mit 1 bis 12 Kohlenstoffatomen oder -D-E ist; R₁₂ und R₁₄ unabhängig voneinander Wasserstoff oder Alkyl mit 1 bis 4 Kohlenstoffatomen sind; und R₁₅ Alkyl mit 1 bis 20 Kohlenstoffatomen ist, wobei D und E die zu Anfang angegebenen Bedeutungen haben.

Ebenfalls von hervorgehobenem Interesse sind schließlich solche Benzofuran-2-one, in denen R₁₁ Phenyl; R₁₃ Alkyl mit 1 bis 4 Kohlenstoffatomen oder -D-E ist; R₁₂ und R₁₄ Wasserstoff sind; und R₁₅ Alkyl mit 1 bis 4 Kohlenstoffatomen, Cyclopentyl oder Cyclohexyl ist, wobei D eine Gruppe -C(R₂₁)₂- und E ein Rest der Formel 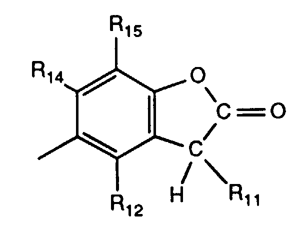 ist, wobei die Substituenten R₂₁ gleich oder verschieden voneinander sind und je Alkyl mit 1 bis 4 Kohlenstoffatomen sind, und R₁₁, R₁₂, R₁₄ und R₁₅ die angegebene Bedeutung haben.

Die Menge an zusätzlichen Additiven, insbesondere Stabilisatoren, z.B. an den genannten Benzofuran-2-onen, kann in weiten Grenzen schwanken. Beispielsweise können sie zu 0,0005 bis 10, vorzugsweise 0,001 bis 5, insbesondere 0,01 bis 2 Gew.-%, in den erfindungsgemäßen Zusammensetzungen enthalten sein.

Die Einarbeitung der Verbindungen der Formel I oder Formel II sowie gegebenenfalls weiterer Additive in das polymere, organische Material erfolgt nach bekannten Methoden, beispielsweise vor oder während der Formgebung oder auch durch Aufbringen der gelösten oder dispergierten Verbindungen auf das polymere, organische Material, gegebenenfalls unter nachträglichem Verdunsten des Lösungsmittels. Die Verbindungen der Formel I oder Formel II können auch in Form eines Masterbatches, der diese beispielsweise in einer Konzentration von 2,5 bis 25 Gew.-% enthält, den zu stabilisierenden Materialien zugesetzt werden.

Die Verbindungen der Formel I oder Formel II können auch vor oder während der Polymerisation oder vor der Vernetzung zugegeben werden.

Die Verbindungen der Formel I oder Formel II können in reiner Form oder in Wachsen, Oelen oder Polymeren verkapselt in das zu stabilisierende Material eingearbeitet werden.

Die Verbindungen der Formel I oder Formel II können auch auf das zu stabilisierende Polymer aufgesprüht werden. Sie sind in der Lage, andere Zusätze (z.B. die oben angegebenen herkömmlichen Additive) bzw. deren Schmelzen zu verdünnen, so dass sie auch zusammen mit diesen Zusätzen auf das zu stabilisierende Polymer aufgesprüht werden können. Besonders vorteilhaft ist die Zugabe durch Aufsprühen während der Desaktivierung der Polymerisationskatalysatoren, wobei z.B. der zur Desaktivierung verwendete Dampf zum Versprühen verwendet werden kann.

Bei kugelförmig polymerisierten Polyolefinen kann es z.B. vorteilhaft sein, die Verbindungen der Formel I oder Formel II, gegebenenfalls zusammen mit anderen Additiven, durch Aufsprühen zu applizieren.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

Wie bereits erwähnt, handelt es sich bei den zu schützenden organischen Materialien vorzugsweise um organische, besonders synthetische, Polymere. Besonders vorteilhaft werden dabei thermoplastische Materialien geschützt, insbesondere Polyolefine. Vor allem ist dabei die ausgezeichnete Wirksamkeit der Verbindungen der Formel I oder Formel II als Verarbeitungsstabilisatoren (Hitzestabilisatoren) hervorzuheben. Zu diesem Zweck werden sie vorteilhaft vor oder während der Verarbeitung des Polymeren diesem zugesetzt. Aber auch weitere Polymere (z.B. Elastomere) oder Schmierstoffe bzw. Hydraulikflüssigkeiten können gegen Abbau, z.B. lichtinduzierten oder thermooxidativen Abbau, stabilisiert werden. Elastomere sind der obigen Aufzählung von möglichen organischen Materialien zu entnehmen.

Die in Frage kommenden Schmierstoffe und Hydraulikflüssigkeiten basieren beispielsweise auf mineralischen oder synthetischen Ölen oder Mischungen davon. Die Schmierstoffe sind dem Fachmann geläufig und in der einschlägigen Fachliteratur, wie beispielsweise in Dieter Klamann, "Schmierstoffe und verwandte Produkte" (Verlag Chemie, Weinheim, 1982), in Schewe-Kobek, "Das Schmiermittel-Taschenbuch" (Dr. Alfred Hüthig-Verlag, Heidelberg, 1974) und in "Ullmanns Enzyklopädie der technischen Chemie", Bd. 13, Seiten 85-94 (Verlag Chemie, Weinheim, 1977) beschrieben.

Eine bevorzugte Ausführungsform der vorliegenden Erfindung ist daher die Verwendung von Verbindungen der Formel I oder Formel II zum Stabilisieren von organischen Materialien gegen oxidativen, thermischen oder lichtinduzierten Abbau.

Bevorzugt werden die erfindungsgemässen Verbindungen der Formel I oder Formel II als Verarbeitungsstabilisatoren (Thermostabilisatoren) von thermoplastischen Polymeren verwendet.

Die vorliegende Erfindung betrifft auch ein Verfahren zum Stabilisieren eines organischen Materials gegen oxidativen, thermischen oder lichtinduzierten Abbau, dadurch gekennzeichnet, daß man diesem mindestens eine Verbindung der Formel I oder Formel II einverleibt oder auf dieses aufbringt.

Die folgenden Beispiele erläutern die Erfindung weiter. Angaben in Teilen oder Prozenten beziehen sich auf das Gewicht.

### Beispiel 1: Herstellung von 2,4-Di-tert-butyl-6-(cyclohexen-1-yl)-phenol (Verbindung (101), Tabelle 1)

24,75 g (120 mMol) 2,4-Di-tert-butylphenol und 2,95 g (30,0 mMol) Cyclohexanon werden bei 60°C eingeschmolzen. Anschliessend wird die Schmelze auf 45°C abgekühlt, mit Salzsäure-Gas gesättigt und während 24 Stunden bei 45°C gerührt. Das Reaktionsgemisch wird mit 50 ml Methanol versetzt und mit Eis/Wasser gekühlt. Das ausgefallene Produkt wird filtriert und mit wenig kaltem Methanol gewaschen. Es resultieren 4,55 g des Produktes. Das Filtrat wird am Vakuumrotationsverdampfer eingeengt und das überschüssige 2,4-Di-tert-butylphenol am Hochvakuum abdestilliert. Kristallisation des Rückstandes aus 10 ml Methanol liefert erneut 1,75 g des Produkts. Es werden somit total 6,3 g (73 %) 2,4-Di-tert-butyl-6-(cyclohexen-1-yl)-phenol, Smp. 102-104°C (Verbindung (101), Tabelle 1) erhalten.

In Analogie zu Beispiel 1 wird ausgehend von 2-tert-Butyl-4-methyl-phenol das 6-(Cyclohexen-1-yl)-2-tert-butyl-4-methyl-phenol (102) hergestellt. Die Reinigung der Verbindung (102) erfolt durch Chromatographie an Kieselgel mit dem Laufmittelsystem Dichlormethan/Hexan = 1:9.

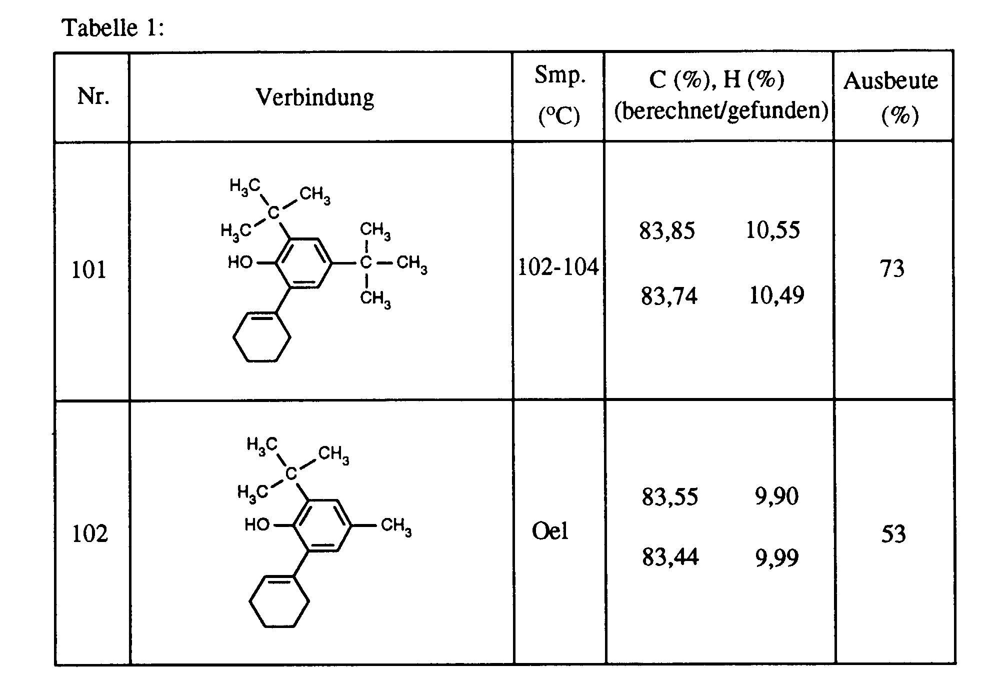

### Beispiel 2: Herstellung von 2-[6-(Cyclohexen-1-yl)-2-tert-butyl-4-methyl-phenoxy]-5,5-dimethyl-1,3,2-dioxaphosphorinan (Verbindung (201), Tabelle 2).

Eine Lösung von 6,6 g (27,0 mMol) 6-(Cyclohexen-1-yl)-2-tert-butyl-4-methyl-phenol (Verbindung (102), Beispiel 1) und 4,0 ml (29,0 mMol) Triethylamin in 25 ml Mesitylen (1,3,5-Trimethylbenzol) werden während ca. 15 Minuten tropfenweise mit 4,85 g (29,0 mMol) 2-Chlor-5,5-dimethyl-1,3,2-dioxaphosphorinan versetzt. Das Reaktionsgemisch wird anschliessend während 18 Stunden unter Rückfluss gekocht, dann abgekühlt, filtriert und am Vakuumrotationsverdampfer eingeengt. Kristallisation des Rückstandes aus trockenem Acetonitril liefert 5,8 g (57 %) 2-[6-(Cyclohexen-1-yl)-2-tert-butyl-4-methyl-phenoxy]-5,5-dimethyl-1,3,2-dioxaphosphorinan, Smp. 97-102°C (Verbindung (201), Tabelle 2).

In Analogie zu Beispiel 2 wird ausgehend von 2,4-Di-tert-butyl-6-(cyclohexen-1-yl)-phenol (Verbindung (101), Tabelle 1, Beispiel 1) die Verbindung (202) (Tabelle 2) erhalten. Mit zwei Aequivalenten 2,4-Di-tert-butyl-6-(cyclohexen-1-yl)-phenol und 3,9-Dichlor-2,4,8,10-tetraoxa-3,9-diphospha-spiro[5,5]undecan anstelle von 2-Chlor-5,5-dimethyl1,3,2-dioxaphosphorinan wird die Verbindung (203) (Tabelle 2) erhalten.

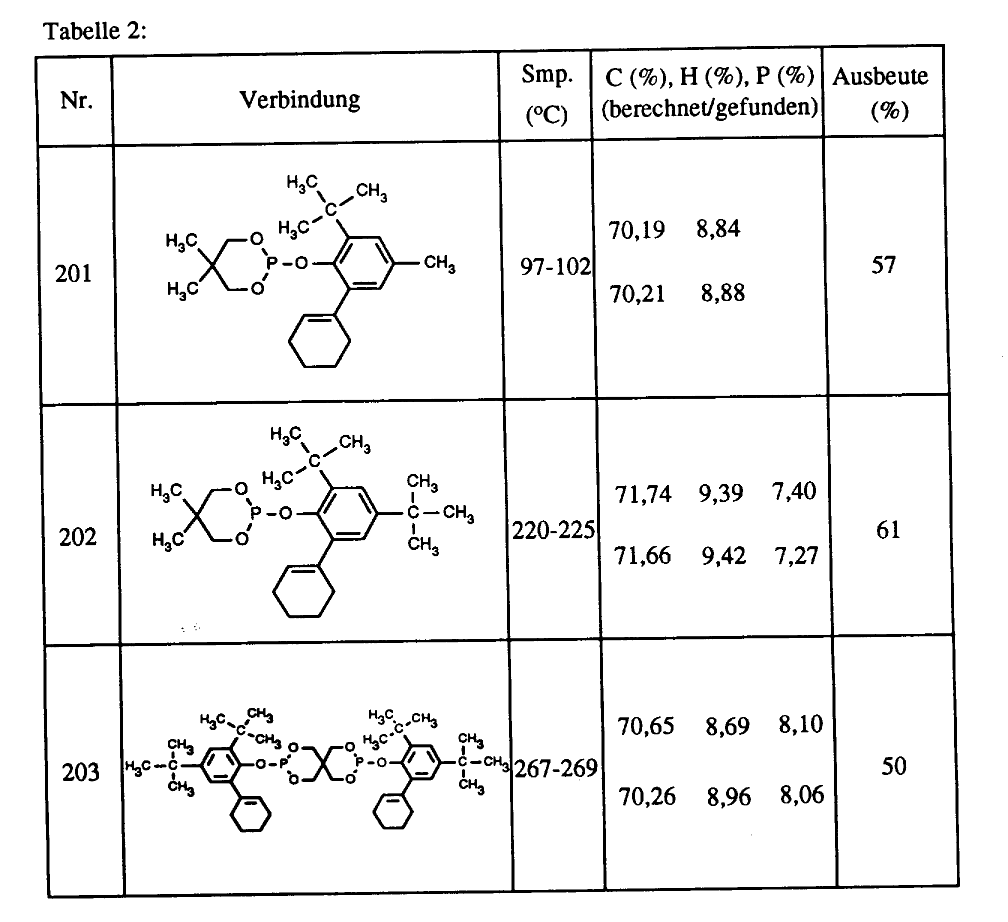

### Beispiel 3: Stabilisierung von Polypropylen bei Mehrfachextrusion.

1,3 kg Polypropylenpulver (Moplen® FL S20), das mit 0,015 % Irganox® 1076 (3-[3,5-di-tert-butyl-4-hydroxyphenyl]propionsäure-n-octadecylester) vorstabilisiert wurde, (mit einem bei 230°C und mit 2,16 kg gemessenen Schmelzindex von 3,2) werden gemischt mit 0,05 % Irganox® 1010 (Pentaerythrit-tetrakis-[3-(3,5-di-tert-butyl-4-hydroxyphenyl)propionat), 0,05 % Calciumstearat, 0,03 % DHT 4A® (Kyowa Chemical Industry Co., Ltd., [Mg_{4.5}Al₂(OH)₁₃CO₃·3,5 H₂O]) und 0,05 % Verbindung aus Tabelle 2. Diese Mischung wird in einem Extruder mit einem Zylinderdurchmesser von 20 mm und einer Länge von 400 mm mit 100 Umdrehungen pro Minute extrudiert, wobei die 3 Heizzonen auf die folgenden Temperaturen eingestellt werden: 260, 270, 280°C. Das Extrudat wird zur Kühlung durch ein Wasserbad gezogen und anschliessend granuliert. Dieses Granulat wird wiederholt extrudiert. Nach 3 Extrusionen wird der Schmelzindex gemessen (bei 230°C mit 2,16 kg). Grosse Zunahme des Schmelzindex bedeutet starken Kettenabbau, also schlechte Stabilisierung. Die Resultate sind in Tabelle 3 zusammengefasst.

**Tabelle 3:**

| Verbindung aus Tabelle 2 | Schmelzindex nach 3 Extrusionen |
|---|---|
| - | 15,0 |
| 201 | 2,80 |
| 202 | 2,90 |
| 203 | 2,60 |

### Beispiel 4: Stabilisierung von Polyethylen während der Verarbeitung.

100 Teile Polyäthylenpulver (Lupolen® 5260 Z) werden mit 0,05 Teilen Irganox® 1010 (Pentaerythrit-tetrakis-[3-(3,5-di-tert-butyl-4-hydroxyphenyl)-propionat] und 0,1 Teilen Stabilisator aus Tabelle 2 gemischt und in einem Brabender Plastographen bei 220°C und 50 Umdrehungen pro Minute geknetet. Während dieser Zeit wird der Knetwiderstand als Drehmoment kontinuierlich registriert. Im Verlauf der Knetzeit beginnt das Polymere nach längerer Konstanz zu vernetzen, was anhand der raschen Zunahme des Drehmoments festgestellt werden kann. In der Tabelle 4 ist die Zeit bis zur merklichen Zunahme des Drehmoments als Mass der Stabilisatorwirkung angegeben. Je länger diese Zeit ist, desto besser ist die Stabilisatorwirkung.

**Tabelle 4**

| Verbindung aus Tabelle 2 | Zeit bis zum Drehmomentanstieg (Min.) |
|---|---|
| - | 5,0 |
| 201 | 11,0 |
| 202 | 10,5 |

## Patentansprüche

1. Verbindungen der Formel I oder II 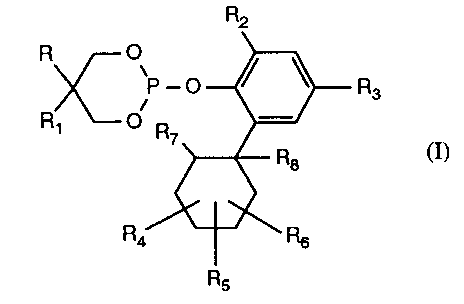 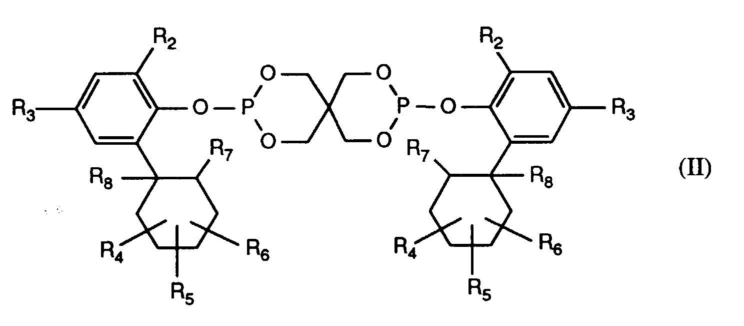 worin
R und R₁ unabhängig voneinander Wasserstoff oder C₁-C₄-Alkyl bedeuten oder zusammen mit dem Kohlenstoffatom, an das sie gebunden sind, einen 3,4-Dehydrocyclohexylidenring bilden,
R₂ Wasserstoff, C₁-C₈-Alkyl oder C₅-C₆-Cycloalkyl darstellt,
R₃ C₁-C₈-Alkyl oder C₅-C₆-Cycloalkyl bedeutet,
R₄, R₅ und R₆ unabhängig voneinander Wasserstoff oder C₁-C₄-Alkyl sind, wobei R₄, R₅ und R₆ zusammen 1 bis 4 Kohlenstoffatome enthalten, und
R₇ und R₈ Wasserstoff bedeuten oder gemeinsam eine zusätzliche direkte Bindung bilden.

2. Verbindungen gemäss Anspruch 1, worin
R₂ Wasserstoff oder C₁-C₄-Alkyl bedeutet,
R₃ C₁-C₄-Alkyl darstellt,
R₄, R₅ und R₆ Wasserstoff sind, und
R₇ und R₈ gemeinsam eine zusätzliche direkte Bindung bilden.

3. Verbindungen gemäss Anspruch 1, worin
R und R₁ Methyl sind,
R₂ und R₃ C₁-C₄-Alkyl bedeuten, und
R₄, R₅ und R₆ Wasserstoff darstellen.

4. Verbindungen gemäss Anspruch 1, worin R₇ und R₈ gemeinsam eine zusätzliche direkte Bindung bilden.

5. Zusammensetzung enthaltend
a) ein dem oxidativen, thermischen oder lichtinduzierten Abbau unterworfenes organisches Material und
b) mindestens eine Verbindung der Formel I oder Formel II gemäss Anspruch 1.

6. Zusammensetzung gemäss Anspruch 5, enthaltend neben den Komponenten (a) und (b) zusätzlich weitere Additive.

7. Zusammensetzung gemäss Anspruch 6, enthaltend als weitere Additive phenolische Antioxidantien, Lichtschutzmittel oder Verarbeitungsstabilisatoren.

8. Zusammensetzung gemäss Anspruch 6, enthaltend als weiteres Additiv mindestens eine Verbindung vom Typ der Benzofuran-2-one.

9. Zusammensetzung gemäss Anspruch 5, enthaltend als Komponente a) natürliche, halbsynthetische oder synthetische Polymere.

10. Zusammensetzung gemäss Anspruch 5, enthaltend als Komponente (a) thermoplastische Polymere.

11. Zusammensetzung gemäss Anspruch 5, enthaltend als Komponente (a) ein Polyolefin.

12. Zusammensetzung gemäss Anspruch 5, enthaltend als Komponente (a) Polyethylen oder Polypropylen.

13. Verwendung der Verbindungen der in Anspruch 1 definierten Formel I oder II als Stabilisatoren für organische Materialien gegen oxidativen, thermischen oder lichtinduzierten Abbau.

14. Verwendung gemäß Anspruch 13, worin die in Anspruch 1 definierten Verbindungen der Formel I oder II als Verarbeitungsstabilisatoren (Thermostabilisatoren) in thermoplastischen Polymeren eingesetzt werden.

15. Verfahren zum Stabilisieren eines organischen Materials gegen oxidativen, thermischen oder lichtinduzierten Abbau, dadurch gekennzeichnet, dass man diesem mindestens eine Verbindung der in Anspruch 1 definierten Formel I oder Formel II einverleibt oder auf dieses aufbringt.

16. Verbindungen der Formel III 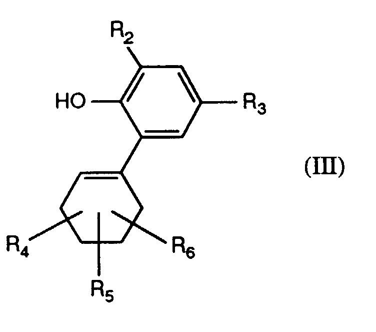
worin R₂ Wasserstoff, C₁-C₈-Alkyl oder C₅-C₆-Cycloalkyl darstellt,
R₃ C₁-C₈-Alkyl oder C₅-C₆-Cycloalkyl bedeutet,
R₄, R₅ und R₆ unabhängig voneinander Wasserstoff oder C₁-C₄-Alkyl sind, wobei R₄, R₅ und R₆ zusammen 1 bis 4 Kohlenstoffatome enthalten, mit der Bedingung, dass die Verbindung der Formel VI ausgeschlossen ist.

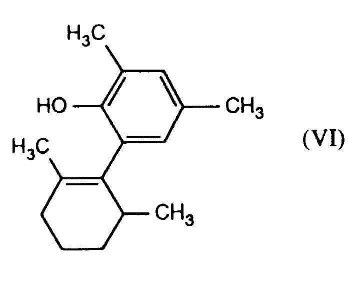

17. Verbindungen gemäss Anspruch 16, worin
R₂ Wasserstoff oder C₁-C₄-Alkyl bedeutet,
R₃ C₁-C₄-Alkyl darstellt, und
R₄, R₅ und R₆ Wasserstoff sind.

18. Verbindungen gemäss Anspruch 16, worin
R₂ tert-Butyl bedeutet.

19. Zusammensetzung enthaltend
a) ein dem oxidativen, thermischen oder lichtinduzierten Abbau unterworfenes organisches Material und
b) mindestens eine Verbindung der Formel III gemäss Anspruch 16.

20. Zusammensetzung gemäss Anspruch 19, enthaltend als Komponente a) natürliche, halbsynthetische oder synthetische Polymere.

## Claims

1. A compound of the formula I or II 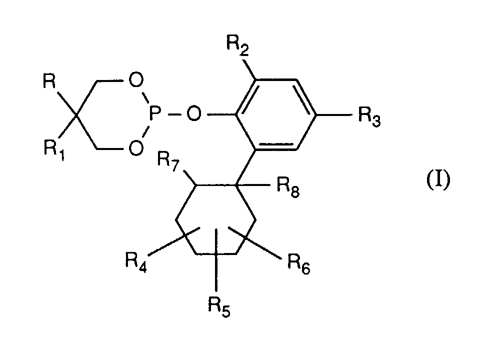 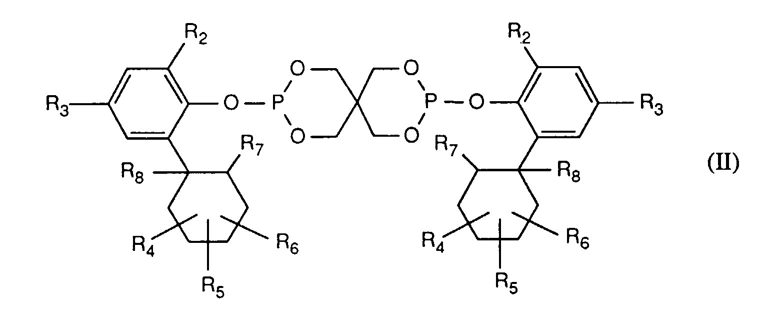 in which
R and R₁, independently of one another, are hydrogen or C₁-C₄alkyl or together with the carbon atom to which they are attached form a 3,4-dehydrocyclohexylidene ring,
R₂ is hydrogen, C₁-C₈alkyl or C₅-C₆cycloalkyl,
R₃ is C₁-C₈alkyl or C₅-C₆cycloalkyl,
R₄, R₅ and R₆, independently of one another, are hydrogen or C₁-C₄alkyl, R₄, R₅ and R₆ together containing 1 to 4 carbon atoms, and
R₇ and R₈ are hydrogen or together form an additional direct bond.

2. A compound according to claim 1, in which
R₂ is hydrogen or C₁-C₄alkyl,
R₃ is C₁-C₄alkyl,
R₄, R₅ and R₆ are hydrogen, and
R₇ and R₈ together form an additional direct bond.

3. A compound according to claim 1, in which
R and R₁ are methyl,
R₂ and R₃ are C₁-C₄alkyl, and
R₄, R₅ and R₆ are hydrogen.

4. A compound according to claim 1, in which R₇ and R₈ together form an additional direct bond.

5. A composition comprising
a) an organic material subjected to oxidative, thermal or light-induced degradation and
b) at least one compound of the formula I or formula II according to claim 1.

6. A composition according to claim 5, additionally comprising, apart from components (a) and (b), further additives.

7. A composition according to claim 6, comprising, as further additives, phenolic antioxidants, light stabilizers or processing stabilizers.

8. A composition according to claim 6, comprising, as the further additive, at least one compound of benzofuran-2-one type.

9. A composition according to claim 5, comprising, as component (a), natural, semi-synthetic or synthetic polymers.

10. A composition according to claim 5, comprising, as component (a), thermoplastic polymers.

11. A composition according to claim 5, comprising, as component (a), a polyolefin.

12. A composition according to claim 5, comprising, as component (a), polyethylene or polypropylene.

13. Use of a compound of the formula I or formula II defined in claim 1 as stabilizer for organic materials against oxidative, thermal or light-induced degradation.

14. Use according to claim 13, in which a compound of the formula I or II as defined in claim 1 is used as processing stabilizer (thermal stabilizer) in thermoplastic polymers.

15. A process for stabilizing an organic material against oxidative, thermal or light-induced degradation, which comprises incorporating therein or applying thereto at least one compound of the formula I or formula II as defined in claim 1.

16. A compound of the formula III 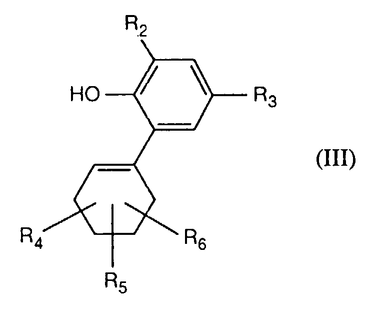 in which
R₂ is hydrogen, C₁-C₈alkyl or C₅-C₆cycloalkyl,
R₃ is C₁-C₈ alkyl or C₅-C₆cycloalkyl,
R₄, R₅ and R₆, independently of one another, are hydrogen or C₁-C₄alkyl, R₄, R₅ and R₆ together containing 1 to 4 carbon atoms, on condition that the compound of the formula VI is excluded.

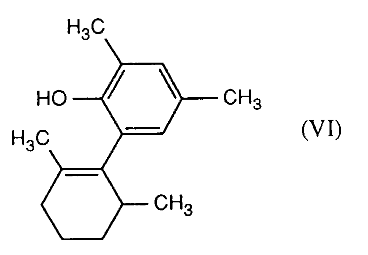

17. A compound according to claim 16, in which
R₂ is hydrogen or C₁-C₄alkyl,
R₃ is C₁-C₄alkyl, and
R₄, R₅ and R₆ are hydrogen.

18. A compound according to claim 16, in which
R₂ is tert-butyl.

19. A composition comprising
a) an organic material subjected to oxidative, thermal or light-induced degradation and
b) at least one compound of the formula III according to claim 16.

20. A composition according to claim 19, comprising, as component a), natural, semi-synthetic or synthetic polymers.

## Revendications

1. Composés de formule I ou II 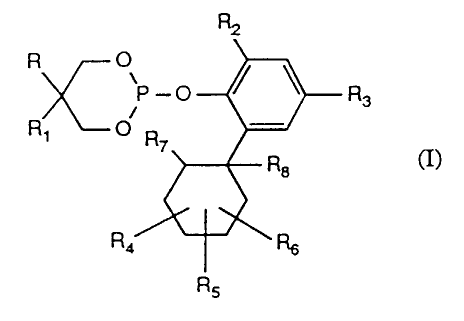 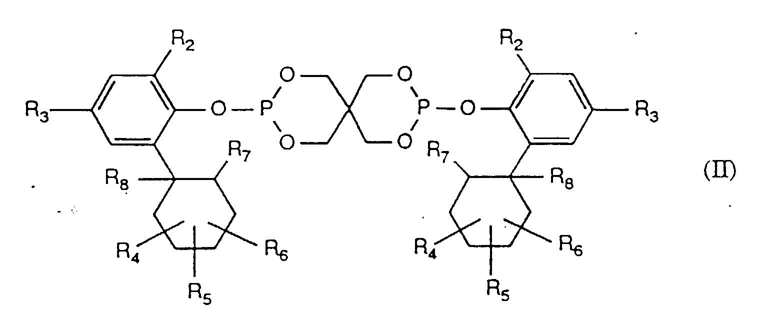
dans lesquelles R et R₁, indépendamment l'un de l'autre, représentent l'hydrogène ou alkyle en C₁-C₄ ou ensemble avec l'atome de carbone auquel ils sont liés forment un cycle 3,4-déshydrocyclohexylidène,
R₂ représente l'hydrogène, alkyle en C₁-C₈ ou cycloalkyle en C₅-C₆,
R₃ représente alkyle en C₁-C₈ ou cycloalkyle en C₅-C₆,
R₄, R₅ et R₆, indépendamment les uns des autres, représentent l'hydrogène ou alkyle en C₁-C₄, R₄, R₅ et R₆ ensemble contenant 1 à 4 atomes de carbone, et
R₇ et R₈ représentent l'hydrogène ou ensemble forment une liaison directe supplémentaire.

2. Composés selon la revendication 1, où
R₂ représente l'hydrogène ou alkyle en C₁-C₄,
R₃ représente alkyle en C₁-C₄,
R₄, R₅ et R₆ sont l'hydrogène, et
R₇ et R₈ ensemble forment une liaison directe supplémentaire.

3. Composés selon la revendication 1, où
R et R₁ sont méthyle,
R₂ et R₃ sont alkyle en C₁-C₄, et
R₄, R₅ et R₆ sont l'hydrogène.

4. Composés selon la revendication 1, où R₇ et R₈ ensemble forment une liaison directe supplémentaire.

5. Composition contenant
a) une matière organique soumise à la dégradation par oxydation, par la chaleur ou par la lumière et
b) au moins un composé de formule I ou de formule II selon la revendication 1.

6. Composition selon la revendication 5, contenant au côté des composants (a) et (b) encore d'autres additifs.

7. Composition selon la revendication 6, contenant comme autres additifs des anti-oxydants phénoliques, des agents photoprotecteurs et des stabilisants de mise en oeuvre.

8. Composition selon la revendication 6, contenant comme autres additifs au moins un composé de type de benzofuran-2-one.

9. Composition selon la revendication 5 contenant comme composant (a) des polymères naturels, semi-synthétiques ou synthétiques.

10. Composition selon la revendication 5, contenant comme composant (a) des polymères thermoplastiques.

11. Composition selon la revendication 5, contenant comme composant (a) une polyoléfine.

12. Composition selon la revendication 5, contenant comme composant (a) le polyéthylène ou le polypropylène.

13. Utilisation des composés de formule I ou II définis dans la revendication 1 comme stabilisant de matières organiques contre la dégradation induite par oxydation, par la chaleur ou par la lumière.

14. Utilisation selon la revendication 13 où les composés de formule I ou II définis dans la revendication 1 sont utilisés comme stabilisants de mise en oeuvre (stabilisants thermiques) dans des polymères thermoplastiques.

15. Procédé pour la stabilisation d'une matière organique contre la dégradation induite par oxydation, par la chaleur ou par la lumière, caractérisé en ce que l'on incorpore à celle-ci ou on applique sur celle-ci au moins un composé de formule I ou de formule II définies dans la revendication 1.

16. Composés de formule III 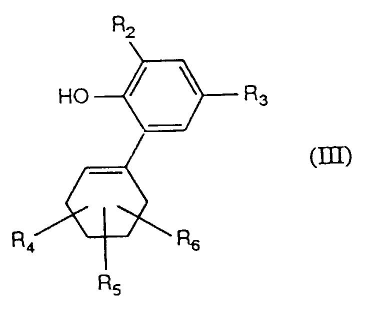 dans laquelle
R₂ représente l'hydrogène, alkyle en C₁-C₈ ou cycloalkyle en C₅-C₆,
R₃ représente alkyle en C₁-C₈ ou cycloalkyle en C₅-C₆,
R₄, R₅ et R₆, indépendamment l'un de l'autre, représentent l'hydrogène ou alkyle en C₁-C₄, R₄, R₅ et R₆ ensemble contiennent 1 à 4 atomes de carbone, à la condition que le composé de formule VI soit exclu.

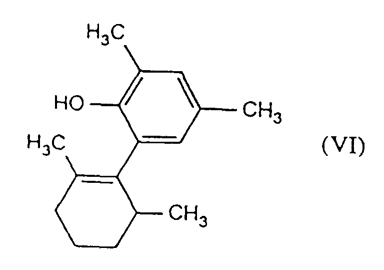

17. Composés selon la revendication 16, où
R₂ représente l'hydrogène ou alkyle en C₁-C₄,
R₃ représente alkyle en C₁-C₄, et
R₄, R₅ et R₆ sont l'hydrogène.

18. Composés selon la revendication 16, où R2 représente tert-butyle.

19. Composition contenant
a) une matière organique soumise à la dégradation induite par oxydation, par la chaleur ou par la lumière et
b) au moins un composé de formule III selon la revendication 16.

20. Composition selon la revendication 19 contenant comme composant a) des polymères naturels, semi-synthétiques ou synthétiques.
